# EUROPEAN PATENT APPLICATION

(11) **EP 4 595 997 A1**
(43) Date of publication of application: **06.08.2025**
(21) Application number: 23874851.1
(22) Date of filing: 03.10.2023
(51) Int. Cl.: A61M 5/142, A61M 5/168, A61F 9/007

(54) **MEDICINE ADMINISTRATION DEVICE**

(30) Priority: 07.10.2022 JP 2022162015
(71) Applicant: Terumo Kabushiki Kaisha, Tokyo 151-0072 (JP)
(72) Inventor: YOSHIKAWA, Hiroki, Ashigarakami-gun, Kanagawa 259-0151 (JP); MITSUOKA, Takumi, Ashigarakami-gun, Kanagawa 259-0151 (JP); SASAKI, Takashi, Ashigarakami-gun, Kanagawa 259-0151 (JP); ARINOBE, Manabu, Ashigarakami-gun, Kanagawa 259-0151 (JP); IWASE, Yoichiro, Ashigarakami-gun, Kanagawa 259-0151 (JP)
(74) Representative: Casalonga
(86) International application number: PCT/JP2023/036052
(87) International publication number: WO 2024/075724

(57) **Abstract**

A drug administration device (10) includes a main body (14) having a cavity (26) therein, a partition wall (16) that partitions the cavity (26) into a first chamber (32) and a second chamber (34), a drug (12) contained in the first chamber (32), a discharge portion (18) connected to the first chamber (32) and configured to discharge the drug (12), a pressure-generating agent (22) contained in the second chamber (34) and expanded by coming into contact with water to push out the partition wall (16) toward the first chamber (32), a semipermeable membrane (20) connected to the second chamber (34) and sealing the pressure-generating agent (22) to the second chamber (34), and a water tank (24) that supplies water to the semipermeable membrane (20).

## Description

### TECHNICAL FIELD

The present invention relates to a drug (medicine) administration device that continuously releases a drug.

### BACKGROUND ART

As a drug administration device that continuously administers a drug, an osmotically-driven drug administration device has been proposed (for example, Japanese Patent No. 4176832). In this drug administration device, the water-swellable substance sealed with a semipermeable membrane swells due to the liquid component that has permeated the semipermeable membrane, and the drug is gradually administered into a living body.

### SUMMARY OF THE INVENTION

The drug administration device described in Japanese Patent No. 4176832 is driven by inflow of water from the semipermeable membrane. Therefore, there is a problem that the drug administration device does not reliably operate in a place where there is little body fluid and it is difficult for water to flow into the semipermeable membrane. For example, in a tissue such as an eye surface, a nasal cavity, an ear (middle ear or inner ear), a digestive tract, an endometrium, an inside of a bone, an inside of a tumor, a lung, a bronchus, or subcutaneous fat, a sufficient amount of water is not supplied, and it is difficult to reliably operate the drug administration device.

Therefore, the conventional osmotically-driven drug administration device has a problem that an indwelling place is limited.

An object of the present invention is to solve the aforementioned problems.

(1) A first aspect of the following disclosure is a drug administration device includes: a main body that has a cavity therein; a partition wall that partitions the cavity into a first chamber and a second chamber; a drug that is contained in the first chamber; a discharge portion that is connected to the first chamber and discharges the drug; a pressure-generating agent that is accommodated in the second chamber, expands by being brought into contact with water, and pushes out the partition wall toward the first chamber; a semipermeable membrane that is connected to the second chamber and seals the pressure-generating agent in the second chamber; and a water tank that supplies water to the semipermeable membrane.

The drug administration device of the above Item (1) can continuously perform the drug administration operation over a long period of time by supplying water from the water tank not only at a location with a body fluid but also at a location without a body fluid or a location with an extremely small amount of a body fluid. Therefore, the drug administration device of Item (1) can be placed at various places.

(2) In the drug administration device according to the above Item 1, the water tank may be configured to cover a portion of the semipermeable membrane exposed from the main body and at least a part of an outer surface of the main body.

Since the drug administration device of the above Item (2) can supply water necessary for operation directly from the water tank to the semipermeable membrane, a pipe connecting the water tank and the semipermeable membrane is unnecessary, and the device configuration is simplified.

(3) In the drug administration device according to the above Item 2, the water tank may be connected to the main body so as to cover the semipermeable membrane in an empty state not containing water in an initial state before use, and the water tank may have an introduction port for introducing water immediately before use.

In the drug administration device described in the above Item (3), since the water tank is empty in the initial state, it is possible to prevent contact between water before the start of use and the semipermeable membrane, it is possible to prevent discharge operation of a drug during transportation and storage, and it is excellent in storage property.

(4) In the drug administration device according to any one of the above Items (1) to (3), the main body and the water tank may have a double pipe structure in which the water tank surrounds an outer peripheral surface of the main body, and one end of the main body provided with the discharge portion may protrude from the water tank.

In the drug administration device according to the above Item (4), since the main body and the water tank have the double tube structure, the total length including the main body and the water tank can be suppressed, and the drug administration device is downsized, so that the drug administration device is easily placed in the living body.

(5) In the drug administration device according to the above Item (1), the water tank may have a partition wall capable of sealing water, and the partition wall can be broken when the water tank is used.

In the drug administration device of the above Item (5), since the water in the water tank and the semipermeable membrane can be separated until immediately before use, the administration operation during storage can be prevented.

(6) In the drug administration device according to the above Item (5), the water tank may contain water in an initial state before use.

In the drug administration device of the above Item (6), water is stored in the water tank in advance. This drug administration device does not require an operation of injecting water into the water tank immediately before use, and thus is excellent in handleability.

(7) In the drug administration device according to the above Item (5), water may be not stored in the water tank in an initial state before use, and the water tank may have an introduction port for introducing water immediately before use.

Since the drug administration device of the above Item (7) can inject water into the water tank immediately before use, there is no need to store water in the tank for a long period of time, and excellent storage performance is achieved.

(8) In the drug administration device according to any one of the above Items (5) to (7), the water tank may be separated from the main body in an initial state before use, the main body may have a holding end where the semipermeable membrane is held, and an attachment portion for mounting the water tank to the holding end.

Since the drug administration device of the above Item (8) can store the water tank containing water in a separated state, contact between water before use and the semipermeable membrane can be effectively prevented, and malfunction during storage can be effectively prevented. Therefore, the drug administration device of Item (8) is also excellent in storage property.

(9) In the drug administration device according to the above Item (8), the semipermeable membrane may have a spike that protrudes from the semipermeable membrane to break the partition wall.

In the drug administration device of the above Item (9), simultaneously with the operation of attaching the water tank to the main body, the breaking operation of breaking the partition wall with the spike can be performed, and the preparation operation before use can be simplified.

(10) In the drug administration device according to any one of the above items (1) to (9), the water tank may have a valve mechanism that allows inflow of a fluid that compensates for consumed water while preventing leakage of water.

The drug administration device described in the above Item (10) can stabilize the supply of water to the semipermeable membrane by preventing the occurrence of the depressurized state of the water tank accompanying the transition of water to the second chamber (consumption of water).

(11) In the drug administration device according to any one of the above items (1) to (10), the water tank may be formed of a flexible material that collapses with consumption of water.

The drug administration device of the above Item (11) can stabilize the supply of water to the semipermeable membrane by preventing the occurrence of the depressurized state of the water tank accompanying the transition of water to the second chamber (consumption of water).

(12) In the drug administration device according to any one of the above Items (1) to (11), the water tank may contain a water-retainable gel.

Since the drug administration device of the above Item (12) stores water in a gel state in the water tank, it is possible to prevent leakage of water from the water tank.

### BRIEF DESCRIPTION OF DRAWINGS

[FIG. 1] Fig. 1 is a cross-sectional view of a drug administration device according to a first embodiment in an initial state.
[FIG. 2] Fig. 2A is an enlarged cross-sectional view of a discharge portion of the drug administration device of Fig. 1, and Fig. 2B is a cross-sectional view taken along line IIB-IIB of Fig. 2A.
[FIG. 3] Fig. 3A is an explanatory view of a method of injecting water into an introduction port of a water tank in the drug administration device of Fig. 1, and Fig. 3B is a cross-sectional view immediately after the start of use of the drug administration device of Fig. 1.
[FIG. 4] Fig. 4A is an explanatory view of a drug administration device according to a first modification of the first embodiment, and Fig. 4B is a cross-sectional view of the drug administration device according to a second modification of the first embodiment.
[FIG. 5] Fig. 5 is a cross-sectional view of a drug administration device according to a third modification of the first embodiment.
[FIG. 6] Fig. 6 is a cross-sectional view of a drug administration device according to a second embodiment in an initial state.
[FIG. 7] Fig. 7A is an explanatory view of a step of attaching a water tank to a main body in the drug administration device of Fig. 6, and Fig. 7B is an enlarged cross-sectional view of the vicinity of an attachment portion of the drug administration device of Fig. 6.

### DESCRIPTION OF THE INVENTION

### (First Embodiment)

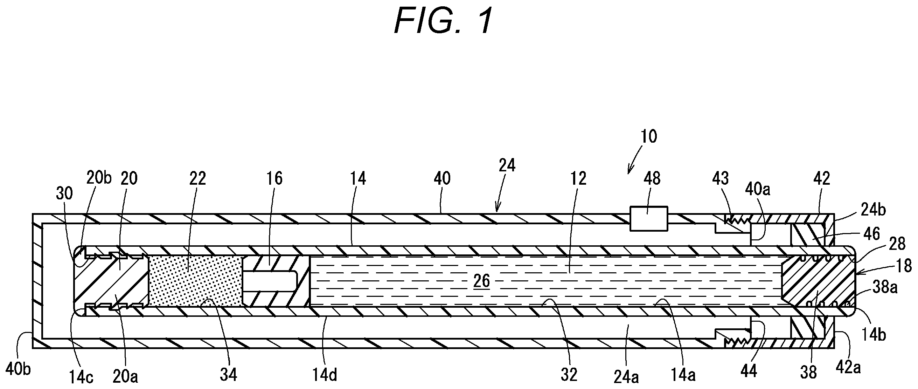

A drug administration device 10 according to the present embodiment illustrated in Fig. 1 is used in an aspect of being embedded in a living body or in an aspect of being attached to a body surface of a living body. The drug administration device 10 can continuously administer a liquid drug 12 contained therein for a relatively long period of time such as several weeks to several years. The drug administration device 10 can reduce the burden on a patient by reducing the frequency of frequent drug administration accompanied by invasion.

The drug administration device 10 includes a main body 14, a partition wall 16, a discharge portion 18, a semipermeable membrane 20, a drug 12, a pressure-generating agent 22, and a water tank 24. Among these, the main body 14 is formed as a straight pipe having a cylindrical shape. The main body 14 has an inner peripheral surface 14a extending in the axial direction with a constant inner diameter. The inner peripheral surface 14a is configured by a smooth curved surface. The main body 14 has a cavity 26 surrounded by the inner peripheral surface 14a. The cavity 26 extends from one end 14b to the other end 14c in the axial direction of the main body 14, opens as a first opening 28 at the one end 14b, and opens as a second opening 30 at the other end 14c.

The main body 14 is formed of a metal material such as stainless steel, a titanium alloy, or an aluminum alloy, or various hard resin materials.

The main body 14 may have a structure in which the partition wall 16 described later is not operated as a piston. In this case, the main body 14 can adopt various shapes capable of containing a desired amount of the drug 12 and the pressure-generating agent 22.

The partition wall 16 is accommodated in the cavity 26. The partition wall 16 is formed of an elastic material such as rubber or elastomer. The partition wall 16 of the present embodiment is configured as a piston. That is, the partition wall 16 can move while sliding on the inner peripheral surface 14a along the axial direction while being in liquid-tight and airtight contact with the inner peripheral surface 14a of the main body 14. The partition wall 16 liquid-tightly and airtightly partitions the cavity 26 into a first chamber 32 on the one end 14b side and a second chamber 34 on the other end 14c side. The partition wall 16 moves toward the one end 14b to reduce the volume of the first chamber 32, and discharges the drug 12 stored in the first chamber 32 from the first chamber 32.

The partition wall 16 is not limited to a piston. In a case where the shape of the main body 14 is not suitable for the operation of the piston, the partition wall 16 may be formed of a flexible membrane.

The discharge portion 18 is located at the one end 14b of the main body 14 and closes the first opening 28. The discharge portion 18 is a cylindrical member accommodated in the cavity 26. The discharge portion 18 includes a plug 38 that abuts on the inner peripheral surface 14a of the main body 14. The plug 38 has a spiral ejection groove 38a on the outer periphery. The ejection grooves 38a allow the outside and the first chamber 32 to communicate with each other. The ejection groove 38a forms a flow path through which the drug 12 flows out between the plug 38 and the inner peripheral surface 14a. Note that the discharge portion 18 is not limited to the above example, and may be a needle tube that punctures a living tissue.

The drug 12 is contained in the first chamber 32. The drug 12 is contained in the first chamber 32 as a liquid (medical solution). The drug 12 is pushed out of the first chamber 32 as the partition wall 16 moves, and is administered into the living body through the discharge portion 18.

The semipermeable membrane 20 is located at the other end 14c of the main body 14 and closes the second opening 30. The semipermeable membrane 20 seals the second chamber 34. The semipermeable membrane 20 has a small diameter portion 20a inserted into the cavity 26 of the main body 14 and a large diameter portion 20b protruding from the other end 14c of the main body 14. The small diameter portion 20a is in close contact with the inner peripheral surface 14a of the main body 14. The large diameter portion 20b has substantially the same diameter as an outer peripheral surface 14d of the main body 14. The large diameter portion 20b is exposed to an internal space 24a of the water tank 24. The semipermeable membrane 20 is formed of a semipermeable membrane material that permeates water while blocking the permeation of the pressure-generating agent 22. Examples of the semipermeable membrane material include plasticized cellulose, hydroxyethyl methacrylate, polyurethane, polyamide, polyether-polyamide copolymer, and thermoplastic copolyester.

The pressure-generating agent 22 is enclosed in the second chamber 34 as a powder (solid) or an aqueous solution. As the pressure-generating agent 22, a substance that expands in volume when coming into contact with water and generates a sufficient pressure inside the second chamber 34 is used. Examples of the pressure-generating agent 22 include common salt (sodium chloride), potassium chloride, magnesium chloride, calcium chloride, superabsorbent polymers, and the like. In view of safety in case of leakage, the pressure-generating agent 22 may employ common salt (sodium chloride). As the pressure-generating agent 22, a substance that reacts with water to generate gas can also be used.

The pressure-generating agent 22 is dissolved in water that has passed through the semipermeable membrane 20 and flowed into the second chamber 34, thereby increasing the volume of the second chamber 34. In a case where the pressure-generating agent 22 is a substance that generates an osmotic pressure, a pressure corresponding to an osmotic pressure difference between the osmotic pressure of the salt concentration in the body and the osmotic pressure of the solution in the second chamber 34 is generated in the second chamber 34. As a result, a pressure difference is generated between the second chamber 34 and the first chamber 32. This pressure difference produces a driving force that displaces the partition wall 16 toward the one end 14b.

The water tank 24 has a cylindrical tank body 40 and a cap 42 concentric with the axis of the main body 14. The tank body 40 and the cap 42 surround the outside of the main body 14. Therefore, the main body 14 and the water tank 24 form a double pipe structure. The water tank 24 has the internal space 24a that contains water between the water tank and the main body 14. In an initial state where a product is provided, no water is stored in the internal space 24a, and the internal space 24a is empty. As an example, the internal space 24a has a volume larger than a volume obtained by adding the volume of the first chamber 32 and the volume of the second chamber 34. Note that the volume of the internal space 24a is not limited to the above example as long as it can contain an amount of water necessary for releasing all or a part of the drug 12 in the first chamber 32.

The tank body 40 and the cap 42 are separably connected by a screw structure 43. The tank body 40 is located on the other end 14c side of the main body 14, and the cap 42 is located on the one end 14b side of the main body 14. The tank body 40 has an introduction port 44 through which water is introduced. In the initial state, the introduction port 44 is closed by the cap 42. When the tank body 40 is removed from the cap 42, the introduction port 44 is exposed.

One end 42a of the cap 42 is located near the one end 14b of the main body 14. The one end 42a of the cap 42 is separated from the one end 14b of the main body 14, and a part near the one end 14b of the main body 14 is exposed to the outside of the water tank 24. That is, the one end 14b of the main body 14 provided with the discharge portion 18 protrudes outward of the water tank 24.

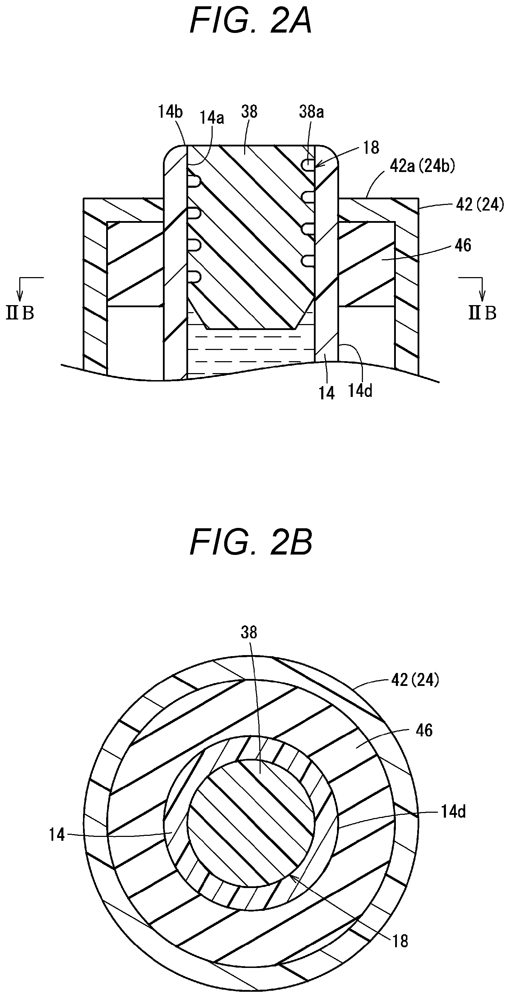

As illustrated in Figs. 2A and 2B, the cap 42 has a packing 46 at the one end 42a. The packing 46 liquid-tightly and airtightly seals a gap between the one end 42a and the outer peripheral surface 14d of the main body 14. The packing 46 prevents leakage of water from a gap between the main body 14 and the water tank 24.

As illustrated in Fig. 1, the water tank 24 further includes a valve mechanism 48. The valve mechanism 48 is constituted by a check valve that prevents leakage of water from the internal space 24a of the water tank 24 and allows inflow of a fluid from the outside of the water tank 24 toward the internal space 24a. When the water tank 24 has a negative pressure, the valve mechanism 48 maintains the internal space 24a of the water tank 24 at normal pressure by allowing air or body fluid to flow in from the outside. The valve mechanism 48 may be formed of a nonwoven fabric sheet that allows passage of air and prevents passage of water. As such a nonwoven fabric sheet, for example, Micropore (registered trademark) manufactured by 3M can be adopted.

The drug administration device 10 of the present embodiment is configured as described above, and its action will be described below together with a usage method.

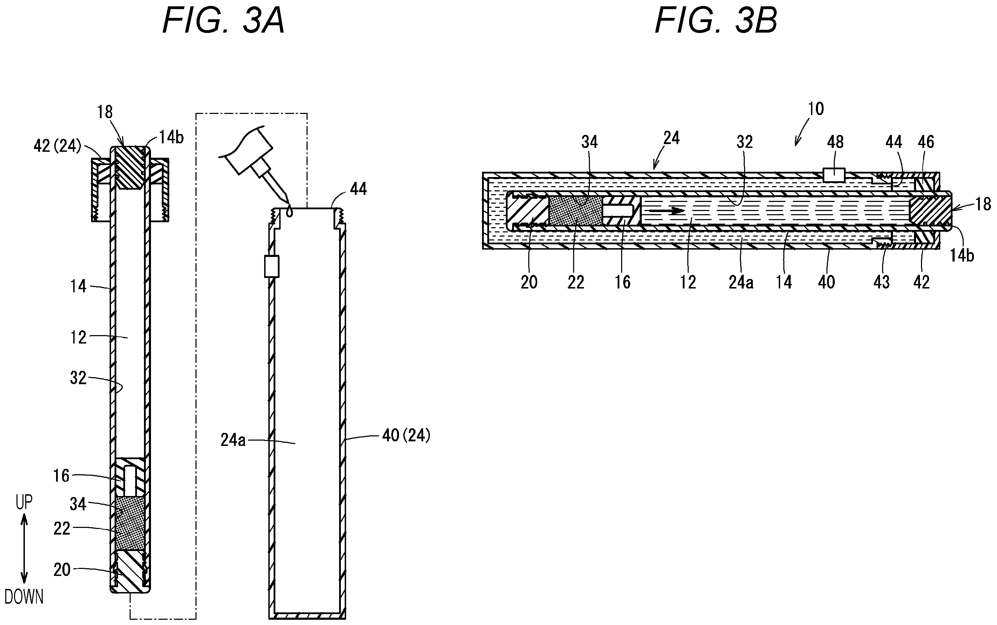

As illustrated in Fig. 3A, in the drug administration device 10, the cap 42 of the water tank 24 and the tank body 40 are separated immediately before use. The tank body 40 is detached from the main body 14 while the cap 42 is attached to the main body 14. The introduction port 44 is exposed to the tank body 40.

Next, as illustrated in the drawing, water is introduced into the tank body 40 through the introduction port 44. Water can be introduced into the tank body 40 using a syringe or the like.

Next, as illustrated in Fig. 3B, the cap 42 is attached to the tank body 40. The cap 42 seals water in the internal space 24a of the water tank 24. With the above operation, the preparation work of the drug administration device 10 is completed.

Thereafter, the drug administration device 10 is placed at a predetermined site inside the living body or on the body surface. In the drug administration device 10, the water sealed in the water tank 24 comes into contact with the semipermeable membrane 20, and moves to the second chamber 34 at a predetermined flow rate set by the dimension of the semipermeable membrane 20 or the like. The water flowing into the second chamber 34 is dissolved or reacted in the pressure-generating agent 22. The pressure-generating agent 22 expands to generate pressure (osmotic pressure) in the second chamber 34. The pressure generated in the second chamber 34 pushes out the partition wall 16 toward the one end 14b of the main body 14, and causes the drug 12 in the first chamber 32 to be discharged from the discharge portion 18.

The discharge speed of the drug 12 can be precisely controlled by the shape of the semipermeable membrane 20. In addition, since the drug administration device 10 of the present embodiment stores water necessary for operation in the water tank 24, it is possible to continuously sustainably release the drug 12 to various sites in the living body where there is no body fluid.

### (First Modification of First Embodiment)

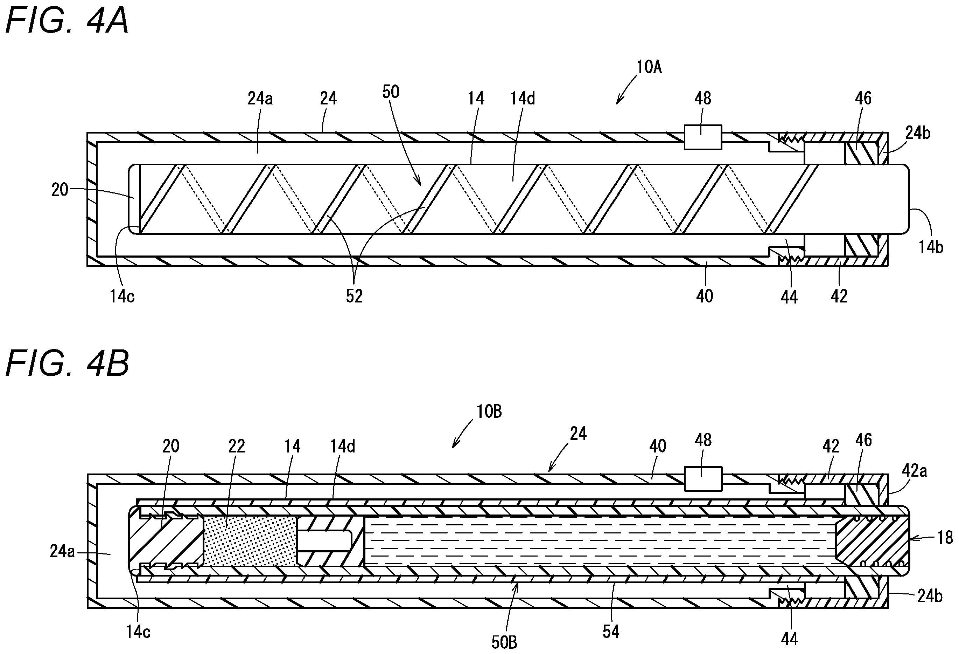

As illustrated in Fig. 4A, a drug administration device 10A of the present modification includes a water transport mechanism 50 in a main body 14. Note that, in the drug administration device 10A of the present modification, configurations other than the water transport mechanism 50 are similar to those of the drug administration device 10 described with reference to Figs. 1 to 3B. Therefore, in the present modification, detailed description of configurations other than the water transport mechanism 50 is omitted.

The water transport mechanism 50 is formed on the outer peripheral surface 14d of the main body 14. The water transport mechanism 50 has a groove 52 formed in a range not overlapping the packing 46 of the water tank 24. The groove 52 is formed in a spiral shape, and an end thereof is connected to the semipermeable membrane 20. The shape of the groove 52 is not limited to a spiral shape, and may be a mesh shape, a linear shape, or the like. The groove 52 constituting the water transport mechanism 50 has a width and a depth at which surface tension of water acts, and guides water in the water tank 24 to the semipermeable membrane 20 by capillary action.

The capillary force (surface tension) increases as the width of the groove 52 is narrowed, and on the other hand, when the width of the groove 52 is excessively narrowed, the amount of water transported decreases. Therefore, the width and depth of the groove 52 are appropriately set according to the administration speed of the drug 12. The width and depth of the groove 52 can be set to, for example, 0.01 to 0.1 mm. In the illustrated example, the groove 52 has a V-shaped cross section. Note that the cross-sectional shape of the groove 52 is not limited to the illustrated example, and may be a rectangular shape or a U-shape. The surface of the groove 52 may be covered with a hydrophilic coating layer. A hydrophilic coating layer can increase the capillary force of the groove 52 to more effectively guide water to the semipermeable membrane 20.

The drug administration device 10A of the present modification can guide water to the semipermeable membrane 20 even in a state where the water in the water tank 24 is consumed and the water is reduced, so that a more stable administration operation can be performed.

### (Second Modification of First Embodiment)

As illustrated in Fig. 4B, a drug administration device 10B of the present modification includes a water transport mechanism 50B in the main body 14. Note that, in the drug administration device 10B of the present modification, configurations other than the water transport mechanism 50B are similar to those of the drug administration device 10 described with reference to Figs. 1 to 3B. Therefore, in the present modification, detailed description of configurations other than the water transport mechanism 50B is omitted.

As illustrated in Fig. 4B, the water transport mechanism 50B has a semipermeable membrane member 54. The semipermeable membrane member 54 is formed of the same material as that of the semipermeable membrane 20. The semipermeable membrane member 54 and the semipermeable membrane 20 may be integrally connected and formed. The semipermeable membrane member 54 extends from the other end 14c of the main body 14 toward the vicinity of the one end 42a of the cap 42 of the water tank 24. However, in order to prevent water leakage, the semipermeable membrane member 54 is disposed inside the water tank 24 with respect to the packing 46. The semipermeable membrane member 54 is bonded to the outer peripheral surface 14d of the main body 14 by a bonding means such as an adhesive.

The semipermeable membrane member 54 can draw water into the semipermeable membrane 20 from a site away from the semipermeable membrane 20 by increasing the surface area of the semipermeable membrane material including the semipermeable membrane 20. Therefore, the drug administration device 10B of the present embodiment can guide the water to the semipermeable membrane 20 even in a state where the water in the water tank 24 is consumed and the water is reduced, so that a more stable administration operation can be performed.

### (Third Modification of First Embodiment)

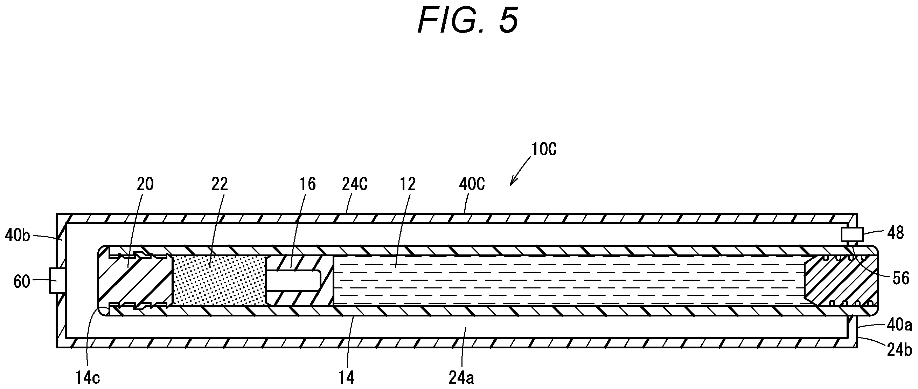

As illustrated in Fig. 5, in a drug administration device 10C of the present modification, a water tank 24C does not have the cap 42, and is formed only of a tank body 40C. Note that, in the drug administration device 10C of the present modification, configurations other than the water tank 24C (tank body 40C) are similar to those of the drug administration device 10 described with reference to Figs. 1 to 3B.
Therefore, in the present modification, detailed description of configurations other than the water tank 24C (tank body 40C) is omitted.

The tank body 40C is formed in a cylindrical shape integrally formed as a whole. Tank body 40C has, at one end 40a, an opening 56 through which the main body 14 passes. The opening 56 is sealed in a liquid-tight and airtight manner. A valve mechanism 48 is provided at the one end 40a of the tank body 40C.

The other end 40b of the tank body 40C covers the outside of the other end 14c of the main body 14. The tank body 40C has a water filling port 60 at the other end 40b. The water filling port 60 is formed of an elastic material through which a needle tube such as rubber can pass. The water filling port 60 can puncture the needle tube of a syringe. The water tank 24C is used by filling the inside of tank body 40C with water through the water filling port 60.

In the drug administration device 10C of the present embodiment as described above, it is not necessary to perform the attaching and detaching operation of the cap 42, and thus, it is possible to simplify the preparation operation before use.

### (Second Embodiment)

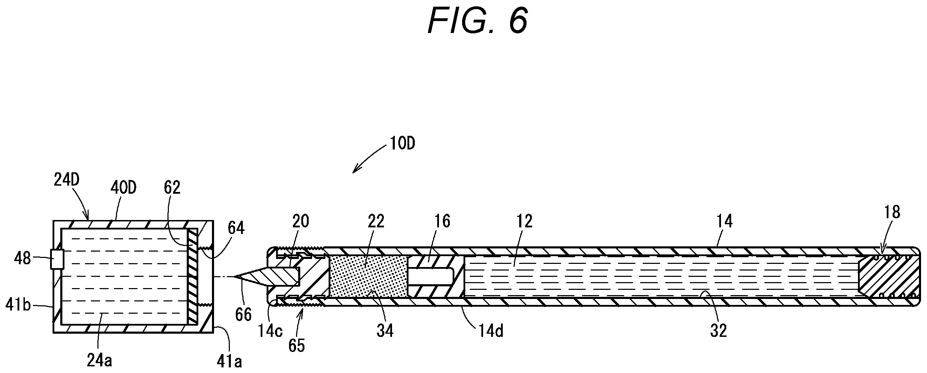

As illustrated in Fig. 6, a drug administration device 10D of the present embodiment has a water tank 24D closed by a partition wall 62 in an initial state. Note that, in the present embodiment, the same components as those of the drug administration device 10 described with reference to Figs. 1 to 3B are denoted by the same reference numerals, and a detailed description thereof will be omitted.

The water tank 24D includes a tank body 40D, a valve mechanism 48, and a partition wall 62. The tank body 40D has a cylindrical shape. Inside the tank body 40D, the internal space 24a for storing water is formed. The tank body 40D has an outer diameter larger than that of the main body 14, and one end 41a of the tank body 40D has a screw structure 64 for fixing to the main body 14. The screw structure 64 is connected to an attachment portion 65 formed on an outer peripheral surface 14d near the other end 14c of the main body 14 by screwing.

The water tank 24D stores water in the internal space 24a closed by the partition wall 62 in the initial state. The water tank 24D can be provided as a product in a state of containing water. The water tank 24D may not store water in the internal space 24a in the initial state. In this case, the water tank 24D may further include an introduction port for introducing water into the water tank 24D immediately before use.

The valve mechanism 48 is located at the other end 41b of the tank body 40D and closes the internal space 24a. The valve mechanism 48 can be a check valve that blocks outflow of water and allows inflow of fluid from the outside. The valve mechanism 48 may be a nonwoven sheet that blocks the passage of water.

The partition wall 62 is located at the one end 41a of the tank body 40D, and closes the internal space 24a of the tank body 40D in a liquid-tight and airtight manner. The partition wall 62 can be broken and is broken when the water tank 24D is attached to the main body 14. Such a partition wall 62 can be made of, for example, a notched rubber material.

On the other hand, the main body 14 of the present embodiment has an attachment portion 65 on the outer peripheral surface 14d near the other end 14c. The screw structure 64 of the water tank 24D is attached to the attachment portion 65. The attachment portion 65 and the screw structure 64 liquid-tightly seal the internal space 24a of the water tank 24.

The semipermeable membrane 20 of the present embodiment has a spike 66 protruding outward. As illustrated in Fig. 6, when the water tank 24D is attached to the main body 14, the spike 66 breaks the partition wall 62 and allows the internal space 24a of the water tank 24D and the semipermeable membrane 20 to communicate with each other.

The drug administration device 10D of the present embodiment is configured as described above. In an initial state in which the drug administration device 10D is provided as a product, as illustrated in Fig. 6, the water tank 24D and the main body 14 are separated from each other.

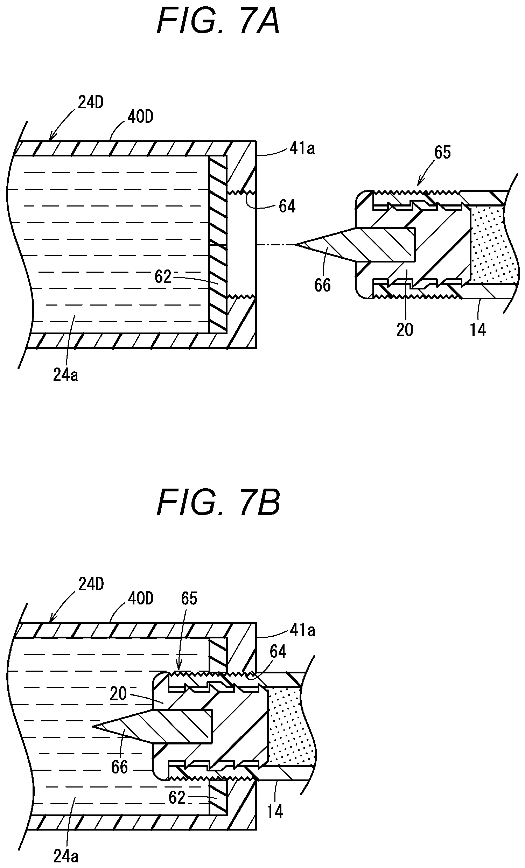

As illustrated in Fig. 7A, the water tank 24D is attached to the main body 14 immediately before use. The partition wall 62 of the water tank 24D is broken by the spike 66 when the water tank 24D is attached to the main body 14. As a result, as illustrated in Fig. 7B, the internal space 24a of the water tank 24D communicates with the semipermeable membrane 20, and the water inside comes into contact with the semipermeable membrane 20. Thereafter, the water tank 24D and the main body 14 are liquid-tightly connected to each other by the screw structure 64 and the attachment portion 65.

Thereafter, the drug administration device 10D is placed at a predetermined position in the living body or on the body surface to administer the drug. As described above, since the drug administration device 10D of the present embodiment can supply water necessary for the operation from the water tank 24D, the administration operation can be performed even when the drug administration device is placed in a place where there is no body fluid.

In addition, the drug administration device 10D of the present embodiment can eliminate the need for preparation work involving a syringe operation, such as introduction of water into the water tank 24D before the start of use.

Note that the present invention is not limited to the disclosure, and various configurations can be adopted without departing from the gist of the present invention.

## Claims

1. A drug administration device comprising:
a main body that has a cavity therein;
a partition wall that partitions the cavity into a first chamber and a second chamber;
a drug that is contained in the first chamber;
a discharge portion that is connected to the first chamber and discharges the drug;
a pressure-generating agent that is contained in the second chamber, expands by being brought into contact with water, and pushes out the partition wall toward the first chamber;
a semipermeable membrane that is connected to the second chamber and seals the pressure-generating agent in the second chamber; and
a water tank that supplies water to the semipermeable membrane.

2. The drug administration device according to claim 1, wherein
the water tank is configured to cover a portion of the semipermeable membrane exposed from the main body and at least a part of an outer surface of the main body.

3. The drug administration device according to claim 2, wherein
the water tank is connected to the main body so as to cover the semipermeable membrane in an empty state not containing water in an initial state before use, and the water tank has an introduction port for introducing water immediately before use.

4. The drug administration device according to claim 2, wherein
the main body and the water tank have a double pipe structure in which the water tank surrounds an outer peripheral surface of the main body, and one end of the main body provided with the discharge portion protrudes from the water tank.

5. The drug administration device according to claim 1, wherein
the water tank has a partition wall capable of sealing water, and the partition wall can be broken when the water tank is used.

6. The drug administration device according to claim 5, wherein
the water tank contains water in an initial state before use.

7. The drug administration device according to claim 5, wherein
water is not stored in the water tank in an initial state before use, and
the water tank has an introduction port for introducing water immediately before use.

8. The drug administration device according to claim **5,** wherein
the water tank is separated from the main body in an initial state before use, the main body has a holding end where the semipermeable membrane is held, and an attachment portion for mounting the water tank to the holding end.

9. The drug administration device according to claim 8, wherein
the semipermeable membrane has a spike that protrudes from the semipermeable membrane to break the partition wall.

10. The drug administration device according to any one of claims 1 to 9, wherein
the water tank has a valve mechanism that allows inflow of a fluid that compensates for consumed water while preventing leakage of water.

11. The drug administration device according to any one of claims 1 to 9, wherein
the water tank is formed of a flexible material that collapses with consumption of water.

12. The drug administration device according to any one of claims 1 to 9, wherein
the water tank contains a water-retainable gel.
